Europäisches Patentamt

European Patent Office

Office européen des brevets

(11) Publication number: **0 335 567**
A2

## EUROPEAN PATENT APPLICATION

(21) Application number: 89302806.8

(22) Date of filing: 21.03.89

(51) Int. Cl.4: **C12N 15/00 , C12N 1/20 ,**
**C12P 21/02 , //(C12N1/20,**
**C12R1:19,1:18,1:425)**

(30) Priority: 30.03.88 US 175111

(43) Date of publication of application:
04.10.89 Bulletin 89/40

(84) Designated Contracting States:
AT BE CH DE ES FR GB GR IT LI LU NL SE

(71) Applicant: BIOGEN, INC.
14 Cambridge Center
Cambridge Massachusetts 02142(US)

(72) Inventor: Fiers, Walter C.
Beukendreef 3
B-9120 Destelbergen(BE)
Inventor: Remaut, Erik R.
Bergstraat 7
B-9221 Vinderhoute(BE)

(74) Representative: Bannerman, David Gardner et
al
Withers & Rogers 4 Dyer's Buildings Holborn
London, EC1N 2JT(GB)

(54) Controlled release of periplasmic proteins into the medium.

(57) This invention relates to methods and intermediates which cause the release of periplasmic proteins of gram-negative bacteria into the growth medium. This invention relates more particularly to novel DNA transfer vectors and gram-negative hosts transformed with them. These vectors are characterized in that they include a DNA sequence encoding a protein, such as kil, which when expressed causes proteins located in the periplasmic space of gram-negative bacteria to be released into the culture medium. It also relates to vectors, transformed hosts and methods for the controlled expression of the kil gene product.

EP 0 335 567 A2

# CONTROLLED RELEASE OF PERIPLASMIC PROTEINS INTO THE MEDIUM

## TECHNICAL FIELD OF INVENTION

This invention relates to methods and intermediates which cause the release of periplasmic proteins of gram-negative bacteria into the growth medium. This invention relates more particularly to novel DNA transfer vectors and gram-negative hosts transformed with them. These vectors are characterized in that they include a DNA sequence encoding a protein, such as kil, which when expressed causes proteins located in the periplasmic space of gram-negative bacteria to be released into the culture medium. It also relates to vectors, transformed hosts and methods for the controlled expression of the kil gene product. As will be appreciated from the disclosure to follow the vectors and transformed hosts of this invention, and the methods of using them, allow for the simplified and cost-efficient recovery of homologous and heterologous polypeptides which, after production in gram negative hosts, are transported to the periplasmic space.

## BACKGROUND OF THE INVENTION

The gram-negative cell wall consists of a plasma membrane, a periplasm and an outer membrane. The plasma membrane is a phospholipid double-layer and contains about 300 different proteins. The peri plasm is a gel which contains diffuse peptidoglycans bound to the outer membrane via lipoproteins. It holds approximately one hundred different proteins. (B. Lugtenberg et al., "Molecular Architecture and Functioning of the Outer Membrane of Escherichia coli and Other Gram-Negative Bacteria", Biochem. Biophys. Acta, 737, pp. 51-115 (1983)).

The outer membrane consists of a mixed glycolipid-phospholipid double-layer which contains some 50 proteins, many of which are receptors for phages and colicines.

The most significant of all of the gram-negative bacteria is E.coli. The advent of recombinant DNA technology has allowed for the expression of many eukaryotic and other heterologous proteins in E.coli. This process has great industrial application in that biologically important proteins can now be produced in much greater quantities than could previously be obtained from natural sources.

The ultimate destination for heterologous, or for that matter homologous, proteins synthesized in E.coli may either be the cytoplasm, the inner membrane, the periplasm or the outer membrane. Very few proteins are naturally secreted across the outer membrane and into the growth medium (D. Muller et al., "Relationship Between Plasmid and Chromosomal Hemolysin Determinants of Escherichia coli", J. Bacteriol, 153, pp. 846-51 (1983)).

Homologous periplasmic proteins are synthesized as precursors in the bacterial cytoplasm and contain a cleavable N-terminal signal sequence which targets them to this subcellular compartment (T. J. Silhavy, "Mechanisms of Protein Localization", Microbiol. Rev., 47, pp. 313-44 (1983)). Eukaryotic proteins synthesized in E.coli have also been directed to the periplasm through recombinant DNA manipulations which fuse the coding region of the protein to a prokaryotic signal sequence. The signal sequences from such periplasmic "carrier" proteins as $\beta$-lactamase (L. Villa-Komaroff et al., "A Bacterial Clone Synthesizing Proinsulin", Proc. Natl. Acad. Sci. U.S.A., 75, pp. 3727-31 (1978)), OmpA protein (J. Ghrayeb et al., "Secretion Cloning Vectors in Escherichia coli", EMBO J., 3, pp. 2437-42 (1983)), and alkaline phosphatase (T. Oka et al., "Synthesis and Secretion of Human Epidermal Growth Factor by Escherichia coli", Proc. Natl. Acad. Sci. U.S.A., 82, pp. 7212-16 (1985)) have all been successfully employed to direct expressed heterologous proteins to the periplasmic space. In some instances the native signal sequence of the eukaryotic protein itself may serve as the periplasm targeting signal (huGH; G. L. Gray et al., "Periplasmic Production of Correctly Processed Human Growth Hormone in Escherichia coli: Natural and Bacterial Signal Sequences are Interchangeable", Gene, 39, pp. 247-54 (1985); United States Patent 4,338,397, EPO Patent 38,182).

Expression of recombinant proteins via secretion into the periplasm offers several advantages over intracellular expression. If the signal sequence is properly cleaved, the heterologous protein will be identical to the naturally occurring product. Periplasmic location may prevent degradation of the protein, as in the case of insulin which was 10-fold more stable in its periplasmic form than in its cytoplasmic form (K. Talmadge et al., "Cellular Location Affects Protein Stability in Escherichia coli", Proc. Natl. Acad. Sci. U.S.A., 79, pp. 1830-33 (1982)). In addition, recombinant periplasmic proteins are more likely to be properly folded due to correct disulphide bond formation. The formation of these bonds has been shown to occur concurrently with secretion across the inner membrane (S. Pollitt et al., "Role of Primary Structure and Disulphide Bond Formation in Beta-Lactamase Secretion", J. Bacteriol., 153, pp. 27-32 (1983)). Proper folding causes the polypeptides produced to be both soluble and biologically active. This has been demonstrated for proinsulin (A. W. Emerick et al., "Expression of a Beta-Lactamase Preproinsulin Fusion Protein in Escherichia coli", Bio/Technology, 2, pp. 165-68 (1984); S. J. Chan et al., "Biosynthesis and Periplasmic Segregation of Human Proinsulin in Escherichia coli", Proc. Natl. Acad. Sci. U.S.A., 78, 5401-05 (1981)) and human EGF (T. Oka et al., Proc. Natl. Acad. Sci. U.S.A., 82, 7212-16). Finally, because periplasmic proteins account for only about 4% of the total cell protein (N. G. Nossal et al., "The Realease of Enzymes by Osmotic Shock from Escherichia coli in Exponential Phase", J. Biol. Chem., 241, pp. 3055-62 (1966)) the requirements of purification are far less extensive than for recombinant proteins that remain in the cytoplasm. For example, high expression of recombinant proteins in the cytoplasm often leads to the formation of insoluble aggregates, termed inclusion bodies. Purification of the desired protein from inclusion bodies involves an initial step of solubilization in a denaturing solvent. Subsequent renaturation may, or may not, lead to full recovery of biological activity.

Prior art purifications of periplasmic proteins involve release of the periplasmic protein fraction by osmotic shock (N. G. Nossal et al., 1966; supra) or by stripping-away the outer cell wall (L. Villa-Komaroff et al., 1978; supra). These techniques have several disadvantages, e.g., introduction of extraneous enzymes which may increase the operation cost and render purification more cumbersome, difficulty of controlling the process (especially on a large scale), separation problems, and risk of partial cell lysis (resulting in a less pure product).

It is therefore, desirable to create a method whereby the advantages of targeting recombinant proteins to the periplasm of transformed bacterial hosts are not negated or lessened by the problems inherent in the recovery and purification of such proteins.

Because the mechanism by which proteins are secreted into the medium by E.coli and other gram-negative bacteria is so poorly understood, little effort has been expended in the development of an expression-excretion system. In one such system the signal sequence and amino-terminus of OmpF protein, a extracellularly secreted protein, was fused to β-endorphin. (K. Nagahari et al., "Secretion into the Culture Medium of a Foreign Gene Product from Escheichia coli: Use of the OmpF Gene for Secretion of Human Beta-Endorphin", EMBO J., 4, 3589-92 (1985)). Over 99% of the β-endorphin was found in the culture medium, but a certain level of cell lysis was present. The mechanism of secretion was not established, but it was hypothesized that the N-terminal of OmpF protein as well as the signal sequence, might play a role in secretion. This mechanism, if true, would preclude excreting a native recombinant polypeptide because a fusion to at least a portion of a mature bacterial polypeptide would be required for secretion into the media.

An alternative to an actual expression-excretion system is a two-step process which involves periplasmic targeting of desired proteins expressed in gram-negative bacteria and release of periplasmic protein into the external media. The kil gene of the ColE1 plasmid encodes a protein which, at low levels, causes the exclusive release of periplasmic proteins without cell lysis.

The kil gene was first identified as a portion of the colicin cassette, which contains the colicin (cea) gene, the immunity (imm) gene and the kil gene, all located in the same operon (J. F. Sabik et al., "cea-kil Operon of the ColE1 Plasmid", J. Bacteriol., 153, pp. 1479-85 (1983)). Expression of the kil gene was hypothesized as being responsible for inhibition of active transport and of protein synthesis, development of abnormal cell permeability, and cell death. Further analysis of the kil gene product was performed by cloning the gene under control of the E.coli lac promoter (M. Altieri et al., "Expression of the Cloned ColE1 kil Gene in Normal and Kil "Escherichia coli", J. Bacteriol., 168, pp. 648-54 (1986)). The transformed strain was viable because it contained an enhanced concentration of lac-repressor protein and was kept at suboptimal temperature. By separating the kil gene from cea and imm, its effects could be monitored independently. It was shown that after 10 minutes of induction of the lac promoter almost 90% of the cells were no longer able to form colonies. The physiological effects of kil were attributed primarily to damage of the cytoplasmic (inner) membrane.

The first indication that the kil gene could cause periplasmic proteins to be excreted was found when the penicillinase gene from Bacillus sp. was cloned into E.coli with pMB9. Upon expression, the majority of penicillinase, a periplasmic protein, was found in the medium (T. Kudo et al., "Excretion of the Penicillinase by Escherichia coli Carrying pEAP2", Eur. J. Appl. Microbiol. Biotechnol., 18, pp. 344-49 (1983)). The secretion of penicillinase was found to be the result of insertional activation of the kil gene caused by the promoter of the inserted penicillinase DNA fragment (T. Kobayashi et al., "Excretion of the Penicillinase of an Alkophilic Bacillus sp. through the Escherichia coli Outer Membrane Is Caused by Insertional Activation of the kil Gene in Plasmid pMB9", J. Bacteriol., 166, pp. 728-32 (1986)).

Attempts to refine this kil-induced excretion system have recently been made. DNA coding for human growth hormone was cloned into a plasmid containing the kil gene which was under control of the weak, constitutive penicillinase promoter. E.coli transformed with this plasmid secreted approx. 55% of the growth hormone into the medium (C. Kato et al., "Construction of An Excretion Vector and Extracellular Production of Human Growth Hormone from Escherichia coli", Gene, 54, pp. 197-202 (1987)).

The present invention reveals the surprising and unexpected result that expression of the kil gene under control of a strong, inducible promoter allows periplasmic proteins to be more rapidly and more efficiently released into the extracellular medium without concommitant cell lysis.

## DISCLOSURE OF THE INVENTION

The present invention solves the problem mentioned above by causing periplasmic proteins to be secreted into the growth medium. Specifically the vectors, hosts and methods of this invention allow for specific and controlled expression of periplasmic release polypeptides, such as kil, by functionally attaching the gene encoding this polypeptide to a strong, inducible promoter. More specifically, the present invention provides the surprising and unexpected result of rapid and efficient release of the periplasmic contents in a transformed cell following induction of the kil gene, with substantially no cell lysis. By virtue of this invention biologically active proteins can be easily recovered from the surrounding media while avoiding the problems inherent in the former methods employed in the release of periplasmic proteins. As will be appreciated from the disclosure to follow the vectors, hosts and methods of the present invention are applicable to the secretion of any protein, both homologous and heterologous to gram-negative bacteria, which is targeted to the periplasm.

## BRIEF DESCRIPTION OF DRAWINGS

Figure 1 displays the location of the 141 nucleotide open reading frame coding for the kil gene between a Tag I site and an XholI site contained within a Bsp1286 restriction fragment from pMB9.

Figure 2 displays the schematic outline of the construction of pPLc321KI and pPLc28KI.

Figure 3 displays the growth curves of induced (closed circles) or non-induced (open circles) E.coli cultures harboring either pPLc321KI or pPLc28KI plasmids. The arrowhead indicates the moment of induction.

Figure 4 displays an SDS-PAGE gel showing the proteins contained in cellular and medium fractions of cultures harboring a plasmid encoding the temperature-sensitive phage repressor, pc1857, alone or in combination with pPLc321KI and grown at inducing or non-inducing temperatures.

Figure 5 displays an autoradiograph of an SDS-PAGE gel of proteins synthesized on a cell-free coupled transcription-translation system using pPLc321KI or pPLc321.

Figure 6 displays the expression cassettes used to produce periplasmic human TNF, including the nucleotide sequence of the fusion point. The fusion point for pPLc245 B-TNF is identical to that of pPLc28-20 B-TNF.

## BEST MODE OF CARRYING OUT THE INVENTION

In order that the invention herein described may be more fully understood, the following detailed description is set forth.

In this description the following terms are employed:

Polypeptide: A linear series of amino acids connected one to the other by peptide bonds between the α-amino and carboxy groups of adjacent amino acids.

Heterologous polypeptide: A polypeptide that is not normally produced by an organism, but is expressed as a result of being transformed with DNA from another source.

Protein: A polypeptide with approximately 50 or more amino acids.

Periplasmic releasing polypeptide: A polypeptide which causes the release into the growth media of periplasmic proteins from gram-negative bacterial cultures, e.g., kil.

DNA Sequence: A linear series of nucleotides connected one to the other by phosphodiester bonds

EP 0 335 567 A2

between the 3′ and 5′ carbons of adjacent pentoses.

Expression: The process undergone by a gene to produce a polypeptide or protein. It is a combination of transcription and translation.

Expression Control Sequence: A DNA sequence that controls and regulates expression of genes when operatively linked to those genes.

Expression Cassette: A DNA sequence consisting of an expression control sequence operatively linked to a polypeptide coding region such that the polypeptide may be expressed in a cell containing the DNA sequence.

Cloning Vehicle or Plasmid: A DNA sequence which is able to reproduce itself in a host cell, characterized by one or a small number of endonuclease recognition sites at which such DNA sequences may be cut in a determinable fashion without attendant loss of an essential biological function of the DNA, and which contains a marker, either before or after transformation, suitable for use in the identification of transformed cells, e.g., tetracycline resistance or ampicillin resistance.

Recombinant DNA Molecule: A hybrid DNA sequence comprising at least two nucleotide sequences, the first sequence not normally being found together in nature with the second.

SOURCE OF KILL GENE

The kil gene was first identified as the component of the ColE1 plasmid responsible for mitomycin-induced lethality and release of colicin (J. F. Sabik et al., J. Bacteriol., 153, pp. 1479-85. (1983)). Thus, the source of the DNA fragment containing the kil gene may be any suitable derivative of ColE1. Many such plasmids are known in the art, such as pMB9 (R. L. Rodriguez et al., In "Molecular Mechanisms in the Control of Gene Expression", D. P. Nierlich et al., eds. ICN-UCLA Symposia on Molecular and Cellular Biology, Vol. V, Academic Press, New York, pp. 471-77 (1976)) and PA02 (A. Oka et al., "Nucleotide Sequence of Small ColE1 Derivatives: Structure of the Regions Essential for Autonomous Replication and Colicin El Immunity", Mol. Gen. Genet., 172, pp. 151-59 (1979)). Removal of the kil-containing fragment can be achieved by digestion with the appropriate restriction endonuclease(s). Choice of restriction enzyme(s) will depend on the vector in which the kil gene is contained and the vector into which the gene will be cloned. These techniques are well-known in the art and are matters of common practice. In the most preferred embodiment of the present invention, the kil gene is excised from pMB9 with Bsp1286 restriction enzyme and further cleaved with either XhoII and TaqI or XhoII and EcoRI to allow for insertion into various vectors.

EXPRESSION CONTROL SEQUENCES

The inducible expression control sequences used in this invention may be chosen from a wide array known in the art. The ability to regulate these sequences is necessary so as to modulate the expression of the kil or other periplasmic release polypeptides and avoid possible lytic effects caused by its overproduction. These sequences may be switched off to enable the host cells to propagate and then switched on to promote expression of the kil gene and subsequent release of the periplasmic contents into the extracellular medium.

Several of these expression control sequences have been employed to express DNA sequences and genes coding for proteins and polypeptides in gram-negative bacterial hosts. These include, for example, the operator, promoter and ribosome binding and interaction sequences of the lactose operon of E.coli (e.g., K. Itakura et al., "Expression in Escherichia coli Of Chemically Synthesized Gene For The Hormone Somatostatin", Science, 198, pp. 1056-63 (1977); D. V. Goeddel et al., "Expression In Escherichia coli of Chemically Synthesized Genes for Human Insulin", Proc. Natl. Acad. Sci. USA, 76, pp. 106-10 (1979), the corresponding sequences of the tryptophan synthetase system of E.coli (J. S. Emtage et al., "Influenza Antigenic Determinants Are Expressed from Haemagglutinin Genes In Escherichia coli", Nature, 283, pp. 171-74 (1980); J. A. Martial et al., "Human Growth Hormone: Complimentary DNA Cloning and Expression in Bacteria", Science, 205, pp. 602-06 (1979)) and the major operator and promoter regions of phage λ (H. Bernard et al., "Construction of Plasmid Cloning Vehicles That Promote Gene Expression from the Bacteriophage Lambda $P_L$ Promoter", Gene, 5, pp. 59-76 (1979); European patent application 41767).

The most preferable embodiment of the present invention utilizes expression control sequences of the temperature-inducible $P_L$ promoter. This promoter is switched off by the temperature-sensitive repressor cl857 at lower growth temperatures. At higher temperatures the repressor is labile and growth of transformants at these temperatures allows the $P_L$ promoter to be switched on, directing synthesis of kil protein. This repressor may be encoded by the same plasmid which contains the kil gene, by a separate plasmid such as pcl857 (E. Remaut et al., "Improved Plasmid Vectors With a Thermoinducible Expression and Temperature Regulated Runaway Replication", Gene, 22, pp. 103-13 (1983)), or on the chromosome of the host as exemplified in the E.coli "pop" strains which contains a non-excisable, defective prophage (e.g., E.coli strains K12ΔHIΔtrp and M5219; European patent application 41767). If a separate plasmid is employed it may be harbored by the host cells of this invention prior to their transformation with the kil-encoding plasmid. Alternatively, the repressor-containing plasmid and the kil-containing plasmid may be used simultaneously to cotransform host cells.

The choice of vector in which to clone the kil gene may be made from a wide variety known in the art. Those vectors which already carry an inducible promoter are preferred, but are not an absolute requirement. The operational linkage of the expression control sequence to the kil gene is the only limitation in the construction of the vectors of this invention. This attachment is achieved through one of several recombinant DNA methodologies routinely used in the art.

In the best mode of the present invention the kil gene is cloned into either pPLc28, which carries the $P_L$ promoter (E. Remaut et al., "Plasmid Vectors for High-Efficiency Expression Controlled by the $P_L$ Promoter of Coliphage Lambda", Gene, 15, pp. 81-93 (1983)), or pPLc321 which contains a modified ribosome-binding site derived from the E.coli trp operon as well as the $P_L$ promoter (Simons et al., "High-Level Expression of Human Interferon Gamma in Escherichia coli Under Control of the $P_L$ Promoter of Bacteriophage Lambda", Gene, 28, pp. 55-64 (1984)). Both vectors have appropriate restriction sites, e.g., EcoRI and BamHI or ClaI and BamHI respectively, into which the kil gene may be properly cloned prior to the transformation of gram-negative bacterial hosts.

## THE HOST CELLS OF THIS INVENTION

Any of a large number of available and well-known gram-negative bacterial host cells may be used in this invention. The selection of a particular host is dependent upon a number of factors recognized by the art. These include, compatibility with the vectors of this invention, sensitivity to the actions of the kil gene product, expression characteristics in regard to both the kil gene and the desired periplasmic protein, biosafety and costs.

With these general guidelines, useful hosts include strains of E.coli, Erwina, Serratia and other gram-negative bacteria (R. Leemans et al., "A Broad Range Expression Vector Based on the $P_L$ Promoter of Bacteriophage Lambda: Reguated Synthesis of Human Interleukin 2 in Erwina and Serratia", J. Bacteriol., 169, pp. 1899-1904 (1987)).

Preferably the host cells of this invention are derivatives of E.coli strain K12 such as MC1061 (pcl857; pPLc28K1), MC1061 (pcl857; pPLc321K1), C3000 (pcl857; pPLc321KI), M5219 (pPLc28KI) or K12ΔH1Δtrp (pPLc28K1 or pPLc321K1).

## PROTEINS PRODUCED BY METHODS OF THIS INVENTION

Any polypeptide or protein that is normally targeted to the periplasm can be secreted into the growth medium by the methods of this invention. These include homologous proteins of the host, heterologous proteins encoded by recombinant DNA harbored by said host (either as a plasmid, prophage or integrated into the host cell chromosome), and fusion protein comprising homologous or heterologous protein or peptide segments. Preferably the proteins produced by the methods of this invention will be of biological and/or medical importance. These include, but are not limited to, human growth hormone, insulin other polypeptide hormones, interferons (e.g. α-interferon, β-interferon and γ-interferon), interleukins (e.g. IL-1 and IL-2), colony-stimulating factors, cytotoxins (e.g., TNF), tissue plasminogen activator, epidermal growth factor, other cell growth factors (e.g. nerve growth factor), and receptors (e.g. T4 receptor and CD4).

Recombinant proteins expressed in gram-negative bacteria can be directed to the periplasm by fusing them to endogenous periplasmic signal sequences at the DNA level (see, e.g., L. VillaKomaroff et al., Proc. Natl. Acad. Sci. USA, 75, pp. 3727-31 (1978); J. Gharyeb et al., EMBO J., 3, pp. 2437-42 (1983); and T. Oka et al., Proc. Natl. Acad. Sci. USA, 82, pp. 7212-16 (1985)). Alternatively, the periplasmic targeting signal may be encoded by synthetic oligonucleotides, hybrid signal sequences or non-endogenous signals.

Examples of the latter include periplasmic signal sequences from other bacteria, various membrane targeting signals from prokaryotic or eukaryotic sources and native signal sequences of the recombinant protein (see e.g., G. L. Gray et al., Gene, 39, pp. 247-54 (1985)). The initial translation product is targeted to the periplasm by the signal sequence, after which the signal is cleaved and the mature protein released into the periplasmic space.

The recombinant plasmids which code for heterologous proteins may be introduced into host cells by transformation prior to or concurrently with the kil-containing plasmid. Alternatively, the DNA encoding the recombinant protein may be contained on the same transfer vector as the kil gene.

In all of the above instances expression of the heterologous protein will also be under control of an expression control sequence. Sequences which may be employed to express these proteins may be selected from either the inducible promoters described above or constitutive promoters which are not under the control of repressors and thus continually promote expression. Several of the latter sequences are known in the art and include the promoters for primer RNA and RNA I, both of which are present in the ColE1 DNA sequence (E. M. Wong et al., "Temperature-Sensitive Copy Number Mutants of ColE1 are Located in an Untranslated Region of the Plasmid Genome", Proc. Natl. Acad. Sci. USA, 79, pp. 3570-74 (1982); Great Britain patent application 8226409), and the promoter of bacteriophage T4 gene 32 (European patent application 241539).

The selected expression control sequence for the heterologous protein should preferably not be the same as the sequences which control expression of kil. By separately regulating the expression of these two products, maximum production of the desired periplasmic protein can be achieved prior to kil-induced secretion into the media. However, the heterologous protein may be expressed concomitantly with kil expression by taking advantage of the slow release of periplasmic proteins after induction in certain bacterial strains such as E.coli strain M5219 harboring the plasmid pPLc28KI.

Proteins produced by the methods and hosts of this invention can be recovered from the media using any of the numerous separation techniques known in the art. Because gram-negative bacteria secrete so few proteins into the media, purification of the desired protein will involve relatively few manipulations. Ammonium sulfate precipitation, gel chromatography, ion-exchange chromatography, affinity chromatography and binding to an immobilized antibody-matrix are just a few of the standard techniques that can be employed to purify the protein of interest. The choice of purification method will depend on the nature of the protein.

In order that this invention may be better understood, the following examples, for illustrative purposes only, are described.


## EXAMPLE 1


### Cloning Of The Kil Gene Under Control Of The $P_L$ Pomoter

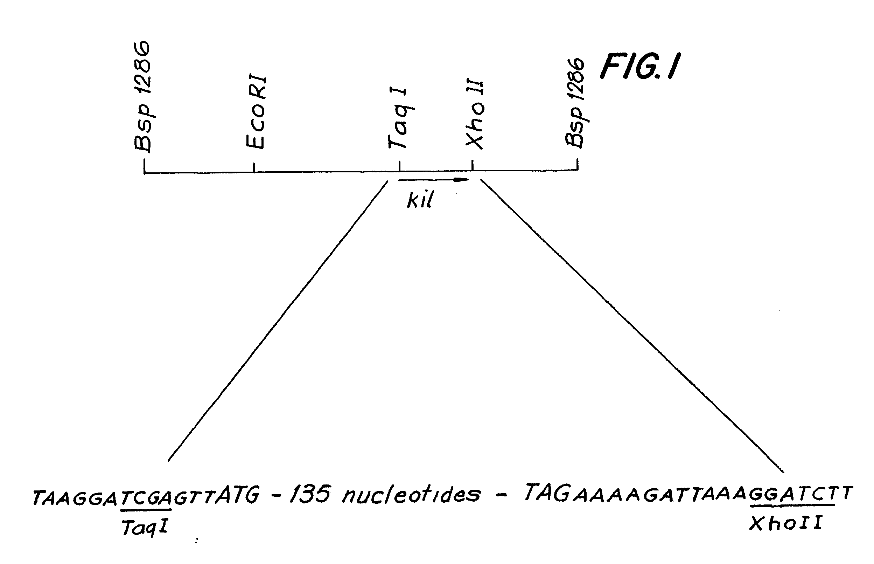

In order to isolate the Bsp1286 fragment of pMB9 containing the kil gene, (see Figure 1) we digested 50 μg of pMB9 DNA with 100 units of Bsp1286 in 500 μl restriction buffer for 4 h at 37°C. The fragments were then separated on an agarose gel and the DNA of interest was isolated by the method of centrifugation-elution (J. Zhu et al., "Construction of Stable Laboratory and Industrial Yeast Strains Expressing a Foreign Gene by Integrative Transformation Using a Dominant Selection System", Gene, 50, pp. 225-37 (1986)). The DNA was then phenol/chloroform extracted and precipitated with 1 volume of isopropanol for 1 hour at -20°C. The DNA was centrifuged and the precipitate was washed with ethanol, dried, and dissolved in 50 μl H2O.

For cloning in pPLc321, we digested 20 μl of the dissolved Bsp1286 fragment with XhoII in a suitable restriction buffer for 3 h at 37°C. The DNA was then phenol/chloroform extracted, ethanol precipitated and transferred to a buffer suitable for restriction with TaqI. This digestion was performed for 3 hours at 65°C.

The pPLc321 vector was digested with ClaI and BamHI for 3 hours at 37°C. After phenol/chloroform extraction and precipitation, the redissolved DNA was mixed with ten-fold molar excess of the kil DNA fragment and ligated at 18°C overnight. As ClaI and TaqI, on the one hand, and Bam HI and XhoII, on the other hand, produce the same sticky ends, the TaqI - XhoII fragment, containing the kil gene, was ligated in the proper orientation following the $P_L$ promoter and the Shine-Dalgarno sequence of pPLc321.

To clone into pPLc28, 20 μl of the dissolved Bsp1286 fragment was again digested with XhoII in a suitable restriction buffer for 3 hours at 37°C. After phenol/chloroform extraction and ethanol precipitation the DNA was transferred to a buffer suitable for restriction with EcoRI. The digestion was carried at 37°C, for 3 hours.

The pPLc28 vector was digested with EcoRI and BamHI for 3 hours at 37°C. After phenol/chloroform extraction and precipitation the redissolved DNA was mixed with a ten-fold molar excess of kil DNA fragment and ligated at 18°C overnight. Because BamHI and XhoII produce the same sticky ends, and the vector contains a unique EcoRI site, the EcoRI - XhoII fragment, containing the kil gene, can be ligated downstream of the $P_L$ promoter in pPLc28.

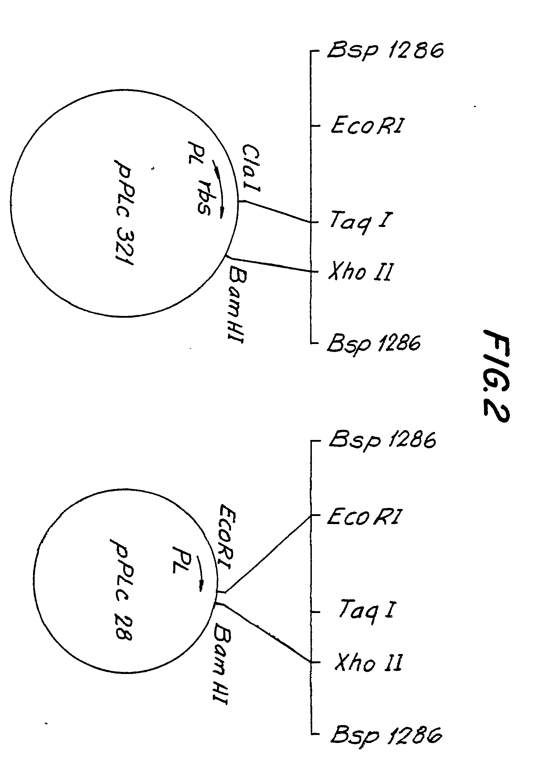

In both cases, transformants were obtained by growing at 28°C in the MC1061(pcI857) strain, following selection for kanamycin and carbenicillin resistance. The constructions of the transformants are shown in Figure 2.

## EXAMPLE 2

### Screening Of Transformants For The Presence Of Kil

It has been reported that 90% of cea⁺, kil⁺ and imm⁺ bearing cells are killed in less than 1 hour after mitomycin C induction. This effect was not observed for cea⁺, kil⁻ and imm⁺ bearing cells. (J. L. Suit et al., "Alternative Forms of Lethality in Mitomycin C-Induced Bacteria Carrying ColE1 Plasmids", Proc. Natl. Acad. Sci. USA, 80, pp. 579-83 (1983)). We used a similar assay to identify kil-bearing transformants except the induction was achieved by a temperature shift up to 42°C. The detection was done by comparing the optical density of samples of the same clone grown at 28°C or 42°C.

20 colonies of the transformants obtained from both the cloning in pPLc321 and pPLc28 were separately grown at 28°C under selective conditions (kanamycin and carbenicillin) in 2 ml LB medium until they reached a density of approx. $2 \times 10^7$ cells/ml (beginning of turbidity). Two 1 ml samples of the different cultures were removed and grown, one at 28°C, the other at 42°. The growth of the cultures was checked each hour by visually comparing the optical density. The colonies showing a growth inhibition at 42°C were regarded as possibly containing the kil gene.

## EXAMPLE 3

### Kinetic Study Of The Growth After Kil Induction

We investigated the kinetics of bacterial growth of the selected MC1061(pcI857; pPLc321KI) and MC1061(pcI857; pPLc28KI) colonies by comparing the changes in optical density before and after induction. These were compared with the growth of MC1061(pcI857) (lacking the kil gene) under similar conditions.

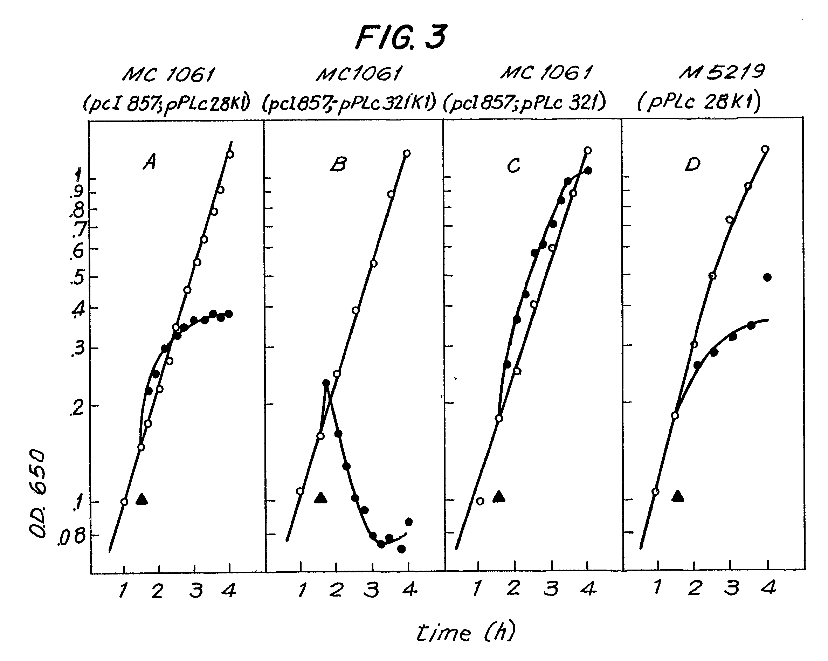

It can be seen from the results shown in Figure 3 that the MC1061(pcI857), MC1061(pcI857; pPLc321K1) and MC1061(pcI857; pPLc28K1) cultures all displayed similar growth kinetics at 28°C. After induction at 42°C, however, the presence of either pPLc28K1 or pPLc321K1 caused a clear growth inhibition. Growth inhibition of the strain containing the pPLc28K1 plasmid was observed after a 60 minute induction. The strain containing pPLc321K1 showed an initial increase in O.D. 650 value which, after 15 minutes, turns into a drop reaching 30% of the maximum value.

In pPLc28K1 the kil gene is situated behind its natural ribosome binding site and is preceded by a DNA stretch in which a transcription termination site appears (P. T. Chan et al., "Nucleotide Sequence and Gene Organization of ColE1 DNA", J. Biol. Chem., 260, pp. 8925-35 (1985)). The latter element might result in a very low transcription of the kil region in pPLc28K1. This hypothesis is supported by our demonstration that cell growth inhibition by pPLc28K1 is more rapidly achieved in a strain expressing the N gene of phage λ, which causes overruling of transcription termination (E. Remaut et al., Gene, 15, pp. 81-93 (1981); A. Marmenout et al., "Oligonucleotide Directed Mutagenesis: Selection of Mutants by Hemimethylation of GATC-sequences", Mol. Gen. Genet., 195, pp. 126-33 (1984)).

## EXAMPLE 4

Cellular Localization Of β-lactamase And β-galactosidase After Kil-Induction

This invention allows for the excretion of periplasmic proteins into the extracellular medium in cells expressing the kil gene under a well controlled and strong promoter. The test system adopted for the assay of excreted periplasmic proteins was the measurement of the β-lactamase activity in the medium. Any occurrence of lysis was checked by measuring the presence of β-galactosidase, a cytoplasmic protein, in the medium.

The cellular localization of β-lactamase and β-galactosidase after kil-induction was investigated in this series of assays. Because the host previously used, MC1061, was lacZ-negative, and thus does not permit a measurement of the β-galactosidase activity, these assays were carried out with the host C3000 (pcl857; pPLc321K1). The C3000 strain was transformed and assayed for expression of the kil gene in an identical manner as done for MC1061.

A culture of C3000 (pcl857; pPLc321K1) was initially grown in LB medium at 28° C until reaching stationary phase. The culture was diluted (1:20) in M9 medium, with lactose as the carbon source and as an inducer of the lac-operon. Part of this culture was shifted to 42° C for induction. Growth of these cultures at 28° C and 42° C̄ was determined at various times after induction.

The assay was performed as follows: 1 ml of each culture grown at the different temperature was centrifuged for 2 minutes. The medium was removed and placed on ice. The cells were transferred to 1 ml LB medium (0° C) and lysed by sonication (5 x 10 sec on ice). The cell debris was removed by centrifugation and the supernatant containing the intracellular proteins was placed on ice -20 µl of both of the medium and lysate fractions of all collected samples were transferred to an Eppendorf tube and incubated at 22° C prior to assaying for β-lactamase and β-galactosidase activity. The reactions were started by adding 100 µl substrate (either ONPG solution for β-galactosidase or PADAC solution for β-lactamase) and shaking well. The reaction was stopped by adding the required stop solution after 25 minutes. and the appropriate O.D. was measured.

Enzymatic activity was measured as follows: β-galactosidase assays -

β-galactosidase assays were performed according to the method given by J. Miller "Experiments in Molelcular Genetics" (Cold Spring Harbor Laboratory, 1982). Enzymatic units were calculated using the following formula:

$$\text{units} = 1000 \times \frac{O.D.420}{O.D.650 \times t \times v}$$

where t = reaction time in minutes;
v = volume of added extract in ml.
O.D.420 = optical density of the reaction mix at 420nm.
O.D.650 = optical density of the bacterial culture at 650nm. prior to the assay.

β-lactamase assays -

β-lactamase assays were performed according to the method of P. Schindler et al., "Use of PADAC, A Novel Chromogenic β-Lactamase Substrate, For the Detection of β-Lactamase Producing Organisms and Assays of β-Lactamase Inhibitors/Inactivators" in Enzyme Inhibitors, V. Brodbeck, ed., Verlag Chemie, Weinheim, pp. 169-76 (1980), using PADAC (Calbiochem Hoechst, Frankfurt, Germany) as the chromogenic substrate. Enzymatic units were calculated using the following formula:

$$\text{units} = 1000 \times \frac{-1 \times O.D.573}{O.D.650 \times t \times v}$$

9

where t = reaction time in minutes;

v = volume of added extract in ml.

0.D.573 = optical density of the reaction mix at 573nm.

0.D.650 = optical density of the bacterial culture at 650nm. prior to the assay Table 1 shows the cellular localization of β-galactosidase at different moments following induction. The cellular localization of β-lactamase is shown in Table 2.

Table 1

| Localization of β-galactosidase after kil-induction by pPLc321K1 in C3000 (pcl857). | | | | | | |
|---|---|---|---|---|---|---|
| t in min | β-galactosidase (units per fraction) | | | | β-galactosidase % intracellular | |
| | 28° C | | 42° C | | 28° C | 42° C |
| | medium | cells | medium | cells | | |
| 0 | 387 | 5032 | - | - | 92.8 | - |
| 30 | 304 | 4694 | 316 | 15166 | 93.9 | 97.9 |
| 60 | 242 | 6303 | 366 | 16274 | 96.3 | 97.8 |
| 90 | 218 | 7558 | 788 | 14660 | 97.2 | 94.8 |
| 120 | 129 | 11102 | 698 | 15230 | 98.8 | 95.6 |
| 150 | 69 | 10912 | 809 | 13231 | 99.3 | 94.2 |

Table 2

| Localization of β-lactamase after kil-induction in pPLc321K1 in C3000(pcl857). | | | | | | |
|---|---|---|---|---|---|---|
| β-lactamase (units per fraction) | | | | | β-lactamase % in the medium | |
| t in min | 28° C | | 42° C | | 28° C | 42° C |
| | medium | cells | medium | cells | | |
| 0 | NM | 3720 | - | - | 0 | - |
| 30 | NM | 4038 | 12726 | 480 | 0 | 96.4 |
| 60 | NM | 4768 | 15422 | 872 | 0 | 94.6 |
| 90 | 118 | 5698 | 18938 | 194 | 2 | 99.0 |
| 120 | 50 | 8042 | 19366 | 56 | 0.6 | 99.7 |
| 150 | NM | 7562 | 18604 | NM | 0 | 100 |
| NM = not measurable. | | | | | | |

The data in Table 2 show that, in the strain C3000 (pcl857; pPLc321K1), 96% of the β-lactamase present is found in the medium fraction within 30 min after kil-induction under the given conditions. As a check on cell lysis we demonstrated that 98% of the β-galactosidase activity was still intracellularly located 30 min post-induction. Thus, the drop in optical density observed for transformants expressing the kil gene is surprisingly not caused by lysis of the bacteria. Microscopic observation has indeed shown that the bacteria clump together after kil induction, which results in a drop of the O.D. 650 value.

This explains the data shown in Tables 1 and 2 which indicate the induced C3000 (pcl857; pPLc321K1) culture contains 3 to 4 times more β-galactosidase and β-lactamase than the control cultures at 28° C. The O.D. 650 value, which is artefactually low due to clumping of bacteria following kil induction, occurs in the denominator of the calculation formula of both the β-galactosidase and β-lactamase units. This results in those values being overestimated. Therefore, the turbidimetric determination at 650 nm is not a suitable parameter to estimate the biomass after the kil-induction. It should be noted that these observations do not affect the calculation of the percentage of β-lactamase released.

The expression of the kil phenotype by means of the pPLc28K1 construction was also tested and its effect on β-lactamase excretion was studied. Initial experiments with pPLc28KI have shown that there is no significant β-lactamase excretion by the usual E.coli strains after kil-induction. As this construction contains a rho-dependent terminator between $P_L$ and kil, the assays were repeated in a strain with transcription antitermination properties. We opted for M5219, because this strain also contains the cI857 gene and codes (chromosomally) for the antitermination N-function of phage λ. Table 3 shows the β-lactamase excretion in this strain. After 150 min induction, 60% of the total β-lactamase is present in the medium.

The E.coli M5219 (pPLc28KI) strain is useful because of its gradual release of periplasmic proteins into the medium without causing a severe growth inhibition. This property allows for the simultaneous induction of kil protein and of a heterologous protein targeted to the periplasm, followed by release of the heterologous protein into the medium. This method may be useful for continuous excretion of periplasmic proteins, e.g., from cells immobilized on a carrier matrix.

Table 3

| Localization of β-lactamase after kil-induction by pPLc28K1 in M5219. | | | | | | |
|---|---|---|---|---|---|---|
| t in min | β-lactamase (units per fraction) | | | | % β-lactamase in the medium | |
| | 28°C | | 42°C | | 28°C | 42°C |
| | medium | cells | medium | cells | | |
| 0 | 408 | | - | - | - | - |
| 30 | 144 | | 1923 | 11261 | - | 14.6 |
| 60 | 53 | | 3940 | 9093 | - | 30.2 |
| 90 | 68 | | 4770 | 6139 | - | 43.7 |
| 120 | 62 | | 4844 | 4849 | - | 50.0 |
| 150 | 59 | | 3716 | 2393 | - | 60.8 |

EXAMPLE 5

Characterization Of The Protein Fraction Released By Kil

The strains C3000(pcI857) and C3000(pcI857; pPLc321KI) were grown in LB medium until reaching stationary phase. Both were inoculated (1:20) into 8 ml of M9 medium + casamino-acids. The cultures were grown in a shaking bath at 28°C until their density reached approx. $6 \times 10^8$ cells/ml. We then transferred 4 ml of each culture to a shaking bath at 42°C for 60 min. The rest of the culture was kept at 28°C. The four different cultures obtained are the following:

1. C3000(pcI857) at 28°C;
2. C3000(pcI857) at 42°C;
3. C3000(pcI857; pPLc321KI) at 28°C;
4. C3000(pcI857; pPLc321KI) at 42°C.

The medium fraction of each culture was isolated as follows: 2 ml of a bacterial culture was transferred to an Eppendorf tube. The cells were removed by centrifugation for 2 min. The supernatant was carefully taken off and TCA was added to a final concentration of 10%. After standing on ice for 10 min. the resulting protein precipitate was collected by centrifugation for 10 min. at 13,000 rpm. The pellet was washed at least four times with 70% ethanol and air-dried afterwards.

The osmotic shockate and cellular fraction were isolated as follows: The pellet of approximately $1.2 \times 10^9$ cells was transferred to 200 μl of a solution consisting of 20% sucrose, 0.1 M Tris - HCl pH 7.6 and 10 mM EDTA. After being kept on ice for 5 min. the cells were collected by centrifuging for 2 min. The supernatant was removed and the pellet was quickly resuspended in 100 μl of ice cold $H_2O$. After 5 min. on ice the cells were collected by centrifuging for 2 min. The supernatant contains the periplasmic protein fraction. The pellet contains the cellular protein fraction.

The medium fraction, the osmotic shockate and the celluar protein fraction of cultures 1, 2 and 3 were collected. From culture 4 only the medium fraction and the cellular protein fraction were collected. Samples were prepared for SDS-PAGE by the addition of SDS-containing sample buffer. 20 μl of the different fractions obtained were analyzed in a 15% polyacrylamide gel. Following electrophoresis the gel was stained with Coomassie Brilliant Blue.

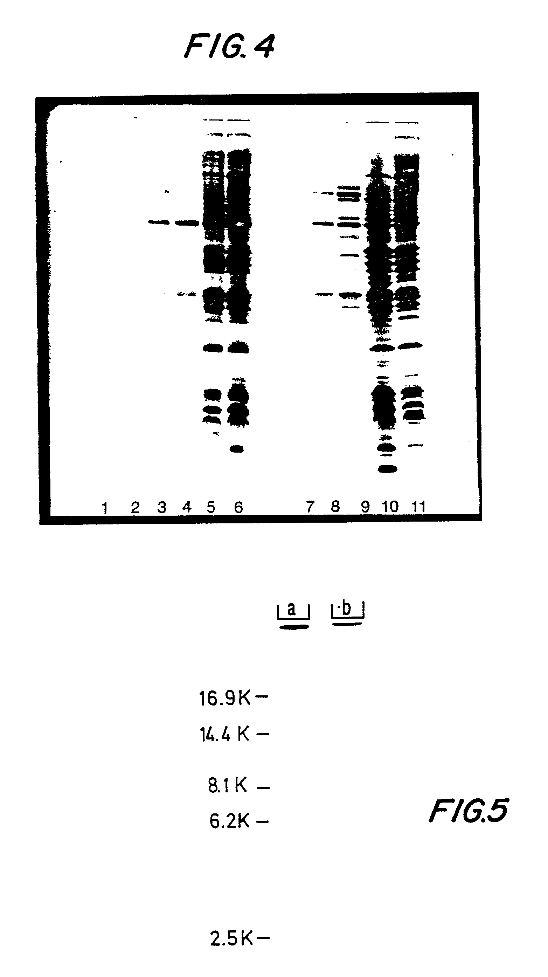

Figure 4 shows that the band pattern of the proteins released into the medium following kil-induction (lane 8) corresponds both quantitatively and qualitatively to the pattern of the proteins which can be released from the periplasm of non-kil bearing cells by osmotic shocking (lane 3).


EXAMPLE 6


Identification Of The Kil Protein

Plasmid-encoded proteins were identified using a cell-free, coupled transcription-translation system (obtained from Amersham International plc, UK). The reactions were performed as recommended by the supplier, using $^3$H-leucine as radioactive amino acid and either pPLc321K1 or pPLc321 as template DNA.

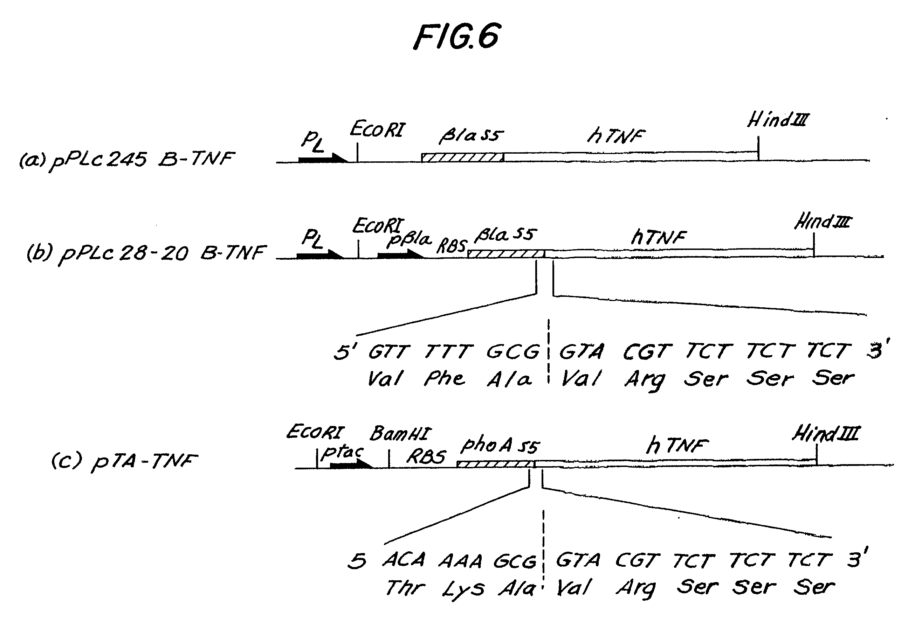

The proteins synthesized were separated on a polyacrylamide gel according to the method of R. T. Swank et al., "Molecular Weight Analysis of Oligopeptides by Electrophoresis in Polyacrylamide Gel with Sodium Dodecyl Sulphate", Anal. Biochem., 39, pp. 462-71 (1971). The gel was treated with EN[3]HANCE (New England Nuclear, Albany, USA) according to the manufacturer's specifications, vacuum-dried and exposed to X-ray film at -70°C. Figure 5 shows the result of this experiment. Plasmid pPLc321K1 directed the synthesis of a polypeptide with a molecular weight of approx. 4 Kd (lane a), which agrees well with the predicted molecular weight of the kil protein calculated from its amino acid sequence. This protein is not synthesized by the control plasmid pPLc321 (lane b).


EXAMPLE 7


A Tumor Necrosis Factor Secretion System: A Two Vector Method

We have constructed two different series of tumor necrosis factor (TNF) secretion vectors, each based on a different expression vector. The first series was based on the Col E1-type vector, the second was based on the RSF1010-type vector. Both types of vector are mobilizable and can be introduced into a wide variety of gram-negative hosts. These types of vectors have been previously described (R. Leemans et al., J. Bacteriol., 169, pp. 1899-1904 (1987)). Into each of these two basic replicons we have inserted three different expression modules (see Figure 6).

Figure 6a shows the first expression cassette, pPLc245 B-TNF. This was based on the left-hand promoter of coliphage lambda, together with the β-lactamase signal sequence. The gene encoding human TNF was fused to the β-lactamase signal sequence. The fusion was such that the C-terminal amino acid fo the signal sequence was in phase with the first amino acid of mature TNF. The $P_L$ promoter is controlled by the temperature-sensitive cI repressor. Induction of the $P_L$ promoter was achieved by a shift in growth temperature from 28°C to 42°C. At 28°C there was virtually no transcription.

The second expression cassette, pPLc28-20 B-TNF, employs the $P_L$ promoter in conjunction with the constitutive β-lactamase promoter. Once again, the human TNF gene was fused in frame to the β-lactamase signal sequence (Figure 6b). This vector was designed to study secretion at various levels of synthesis and induction as well as at various temperatures. When either of the above two expression vectors were used the temperature-sensitive cI function was provided from either a compatible plasmid or a defective lambda prophage on the transformed cell chromosome.

The final expression cassette, pTA-TNF, was created by fusing the TNF gene to the alkaline phosphatase signal sequence. Expression of the fusion protein was under control of the IPTG-inducible tac promoter (Figure 6c). In all of these expression vectors the TNF fusion proteins were targeted to the periplasm of transformed cells by the corresponding signal sequence and the signal subsequently cleaved releasing mature recombinant TNF.

The RSF1010-derived plasmids are already compatible with the kil-containing plasmids pPLc28K1 and pPLc321K1. When utilizing this combination transformants can be selected by both kanamycin (TNF-containing plasmid) and ampicillin (kil-containing plasmid) resistance. Thus, cotransformation or sequential transformation of a gram-negative host with one of the kil-containing plasmids and one of the TNF-encoding plasmids will create a system whereby mature TNF is excreted into the growth medium under appropriate conditions of induction.

## EXAMPLE 8

### A Tumor Necrosis Factor Secretion System: A Single Plasmid Method

A single plasmid is constructed which contains the TNF gene linked to a periplasmic signal sequence and under control of the tac promoter. The plasmid also contains the kil gene under control of the $P_L$ promoter. The vector is used to transform a gram-negative bacterial host. This construct allows an uncoupling of kil induction (42° C) and TNF induction (IPTG). Such a system facilitates the optimization of this selective secretion system in E.coli, as well as in other gram-negative organisms.

Microorganisms prepared by the processes of this invention are exemplified by cultures deposited in the In Vitro International, Inc. culture collection, in Linthicum, Maryland, on March 11, 1988 (for cultures 1798 and 1824) and on March 17, 1988 (for cultures 1481 and 1823) and identified as:

1798: E.coli K514-pcl857, pPLc-28-20BTNF

1824: E.coli M5219-pPLc28K1

1481: E.coli MC1061-pcl857, pPLc321K1

1823: E.coli C3000-pcl857, pPLc321K1

These cultures were assigned accession numbers IVI 10163-10166, respectively.

While we have hereinbefore described a number of embodiments of this invention, it is apparent that our basic constructions can be altered to provide other embodiments which utilize the vectors, hosts and methods of this invention. Therefore, it will be appreciated that the scope of this invention is to be defined by the claims appended hereto rather than by specific embodiments which have been presented hereinbefore by way of example.

## Claims

1. A recombinant DNA molecule comprising a DNA sequence coding for a periplasmic releasing polypeptide and being operatively linked in the recombinant DNA molecule to an inducible expression control sequence selected from the group consisting of $P_L$, $P_R$ and derivatives thereof.

2. The recombinant DNA molecule according to claim 1, wherein said periplasmic releasing polypeptide is the kil protein.

3. The recombinant DNA molecule according to claim 2, selected from the group consisting of pPLc28K1, pPLc321K1, and derivatives thereof.

4. A recombinant DNA molecule comprising a first DNA sequence coding for a periplasmic releasing polypeptide and being operatively linked in the recombinant DNA molecule to an inducible expression control sequence and a second DNA sequence coding for a polypeptide targeted to the periplasm of gram-negative bacterial cells and being operatively linked in the recombinant DNA molecule to an expression control sequence selected from the group consisting of expression control sequences that are identical to and expression control sequences that are different from the inducible expression control sequence operatively linked to the first DNA sequence.

5. The recombinant DNA molecule according to claim 4, wherein the periplasmic releasing polypeptide is the kil protein.

6. The recombinant DNA molecule according to claim 4, wherein the polypeptide targeted to the periplasm is selected from the group consisting of growth hormones, insulins, interferons, interleukins, colony-stimulating factors, tissue plasminogen activators, T-cell receptors, cytotoxins, epidermal growth factor, and other growth factors.

7. The recombinant DNA molecule according to claim 4, wherein the first and second DNA sequences are operatively linked to identical expression control sequences.

8. The recombinant DNA molecule according to claim 4, wherein the first and second DNA sequences are operatively linked to a single expression control sequence.

9. The recombinant DNA molecule according to claim 4, wherein the first and second DNA sequences are operatively linked to different expression control sequences.

10. The recombinant DNA molecule according to claim 4, wherein the first DNA sequence is operatively linked to an inducible expression control sequence selected from the group consisting of $P_L$, $P_R$, and derivatives thereof.

11. A gram-negative bacterial strain comprising a first DNA sequence coding for a periplasmic releasing polypeptide and being operatively linked to an inducible expression control sequence, and a second DNA sequence coding for a heterologous polypeptide targeted to the periplasm and being operatively linked to an expression control sequence selected from the group of expression control sequences that are identical to and expression control sequences that are different from the inducible expression control sequence operatively linked to the first DNA sequence.

12. The bacterial strain according to claim 11, wherein the first DNA sequence is located on the bacterial chromosome or on a first recombinant DNA molecule and the second DNA sequence is located on the bacterial chromosome or on a second recombinant DNA molecule.

13. The bacterial strain according to claim 11 or 12, selected from the group consisting of E.coli, Erwinia, and Serratia.

14. The bacterial strain according to claim 13 wherein the strain is a derivative of E.coli selected from the group consisting of MC1061 (pcl857; pPLc28K1), MC1061 (pcl857; pPLc321K1), C3000 (pcl857; pPLc28K1), and M5219 (pPLc28K1),

15. A gram negative bacterial strain transformed with a recombinant DNA molecule according to any of claims 1-10.

16. A method for producing a polypeptide comprising the steps of:

(a) culturing a bacterial strain according to any of claims 11-15 under growth conditions which do not induce expression of the DNA sequence coding for periplasmic releasing polypeptide; and

(b) altering the growth conditions so as to induce expression of the DNA sequence coding for periplasmic releasing polypeptide.

17. The method according to claim 16, wherein the growth conditions of step (a) permit expression of the polypeptide targeted to the periplasm.

18. The method according to claim 17, further comprising an initial step of culturing the bacterial strain under growth conditions which do not induce expression of the polypeptide targeted to the periplasm and do not induce expression of the periplasmic releasing polypeptide.

19. The method according to claim 16 wherein the bacterial strain is cultured under growth conditions which both induce expression of the DNA sequences coding for the periplasmic releasing polypeptide and permit the expression of polypeptide targeted to the periplasm.

20. The method according to any of claims 16-19, further comprising the step of isolating the polypeptide targeted to the periplasm from the growth medium.

21. The method according to claim 20, wherein the polypeptide targeted to the periplasm is selected from the group consisting of growth hormones, insulins, interferons, interleukins, colony stimulating factors, tissue plasminogen activators, T-cell receptors, cytotoxins, epidermal growth factors and other growth factors.

TAAGGATCGGAGTTATG - 135 nucleotides - TAGAAAAGGATTAAAGGATCTT

Taq I

Xho II

Bsp 1286

EcoRI

Taq I

kil

Xho II

Bsp 1286

FIG.1

FIG.2

FIG.3

# FIG. 4

1   2   3   4   5   6       7   8   9 10  11

|a|     |·b|

16.9K −

14.4 K −

8.1 K −

6.2 K −

# FIG.5

2.5K −

# FIG.6

(a) pPLc 245 B-TNF

PL  | EcoRI  Bla ss  hTNF  | HindIII

(b) pPLc 28-20 B-TNF

PL  | EcoRI pβla RBS Bla 55  hTNF  | HindIII

5' GTT TTT GCG | GTA CGT TCT TCT TCT 3'
   Val Phe Ala | Val Arg Ser Ser Ser

(c) pTA-TNF

ptac  | EcoRI BamHI phoA 55  hTNF  | HindIII
       RBS

5 ACA AAA GCG | GTA CGT TCT TCT TCT TCT 3'
  Thr Lys Ala | Val Arg Ser Ser Ser Ser